(19) Europäisches Patentamt
European Patent Office
Office européen des brevets

(11) Publication number: 0 106 571 A2

(12) # EUROPEAN PATENT APPLICATION

(21) Application number: 83305605.4

(22) Date of filing: 21.09.83

(51) Int. Cl.³: A 61 K 31/557
A 23 L 2/38, A 23 L 2/26
A 23 L 1/30

(30) Priority: 22.09.82 ZA 826965

(43) Date of publication of application: 25.04.84 Bulletin 84/17

(84) Designated Contracting States: AT BE CH DE FR GB IT LI LU NL SE

(71) Applicant: SENTRACHEM LIMITED
P.O. Box 61204
Marshalltown Transvaal 2107(ZA)

(72) Inventor: Booyens, Jakobus
Plot 45
Patryshoek(ZA)

(74) Representative: Frankland, Nigel H. et al,
FORRESTER & BOEHMERT Widenmayerstrasse 4/I
D-8000 München 22(DE)

(54) **Prophylactic substance or composition.**

(57) The invention concerns a method of preventing the multiplication of viable cancer cells by the regular administration of a prophylactic amount of a precursor for the production, in vivo of one or more of the following eicosanoid mixtures

| a | b | c |
|---|---|---|
| $PGE_1$ | $PGE_2$ | $PGE_3$ |
| $PGF_1$ | $PGF_2$ | $PGF_3$ |
| $TXA_1$ | $PGD_2$ | $PGD_3$ |
| | Prostacyclin | $\Delta^{17}$Prostacyclin |
| | $TXA_2$ | $TXA_3$ |
| $LTA_3$ | $LTA_4$ | $LTA_5$ |
| $LTC_3$ | $LTB_4$ | $LTB_5$ |
| $LTD_3$ | $LTC_4$ | |
| | $LTD_4$ | |
| | $LTE_4$ | |

and particularly GLA, AA and/or EPA.

EP 0 106 571 A2

Croydon Printing Company Ltd.

Title: "Prophylactic Susbstance or Composition"

THIS INVENTION relates to substances and compositions containing such substances for use in the prophylaxis of cancerous conditions.

In 1980 Horrobin (The Reversibility of Cancer: The Relevance of Cyclic AMP, Calcium, Essential Fatty Acids and Prostaglandin $E_1$ Med. Hypotheses 1980, Vol. 6, pages 469 to 486) dealt extensively with metabolic abnormalities common to almost all cancer cells, and also discussed possible causative factors for these. Horrobin concludes that a metabolic abnormality in the synthesis of the prostaglandins thromboxane $A_2$ ($TXA_2$) and prostaglandin $E_1$ ($PGE_1$) is the final factor which allows an initiated cancer cell to express its abnormality, that is to divide ad infinitum. Horrobin further proposed (on the basis of evidence present in the general literature) that the defect which leads to the abnormality in the synthesis of $TXA_2$ and $PGE_1$ is an inhibition of the enzyme delta-6-desaturase. This enzyme converts the essential fatty acid linoleic acid (LA), to gamma linolenic acid (GLA) in all normal cells of the body. GLA is further metabolised to dihomo-gamma-linolenic acid (DGLA) which in turn is converted to prostaglandins of the 1-series, which includes $PGE_1$.

$PGE_1$ and $TXA_2$ are potent intracellular regulators of the biochemistry of all normal cells. $TXA_2$ however cannot function in the absence of $PGE_1$. Horrobin surmised that a deficiency of $PGE_1$ and thus disabled $TXA_2$, will cause an abnormal metabolism of the cell, of sufficient magnitude to trigger off uncontrolled division of potential cancer cells.

Horrobin thus proposed that inter alia a GLA supplement should be given to cancer patients receiving conventional treatment, in order to test his hypothesis, namely, that in bypassing the metabolic block caused by an inhibited delta-6-desaturase (d-6-d) activity, it should be possible to normalise cancer cells by reverse transformation.

In European Patent Publication No. 0 037 Horrobin discloses a mixture of GLA and thioproline for the treatment of cancer.

According to one aspect of the invention there is provided a prophylactic method for the prevention of the multiplication of viable cancer cells includes the step of administering, on a regular basis, to a human or animal, a prophylactic amount of one or more precursors for the production in vivo, of one or more of the following eicosanoid mixtures, namely

| a | b | c |
|---|---|---|
| $PGE_1$ | $PGE_2$ | $PGE_3$ |
| $PGF_1$ | $PGF_2$ | $PGF_3$ |
| $TXA_1$ | $PGD_2$ | $PGD_3$ |
| | Prostacyclin | $\Delta^{17}$Prostacyclin |
| | $TXA_2$ | $TXA_3$ |
| $LTA_3$ | $LTA_4$ | $LTA_5$ |
| $LTC_3$ | $LTB_4$ | $LTB_5$ |
| $LTD_3$ | $LTC_4$ | |
| | $LTD_4$ | |
| | $LTE_4$ | |

according to the following scheme

BAD ORIGINAL

Elongase — $\Delta^5$ Desaturase

$C_{18:3}, \omega 6$ → $C_{20:3}, \omega 6$ → $C_{20:4}, \omega 6$

γ-Linolenic — Dihomo-γ-linolenic — Arachidonic

From Dihomo-γ-linolenic:

$PGE_1$
$PGF_1$
$TXA_1$

$LTA_3$
$LTC_3$
$LTD_3$

From Arachidonic:

$PGE_2$
$PGF_2$
$PGD_2$
Prostacyclin
$TXA_2$

$LTA_4$
$LTB_4$
$LTC_4$
$LTD_4$
$LTE_4$

$C_{18:4}, \omega 3$ → $C_{20:4}, \omega 3$ → $C_{20:5}, \omega 3$

Ostadeca-tetraenioc — Eicosa-tetraenoic — Eicosa-pentaenoic

From Eicosapentaenoic:

$PGE_3$
$PGF_3$
$PGD_3$
$\Delta^{17}$ Prostacyclin
$TXA_3$

$LTA_5$
$LTB_5$
$LTC_5$

BAD ORIGINAL

In the preferred form of the invention the precursor is chosen from GLA, AA and EPA, or salts, derivatives and analogues thereof.

Such administration does not involve any toxic or other harmful side effects (for example the compounds do not affect the growth of benign cells).

According to another aspect of this invention there is provided a prophylactic composition for the prevention or minimisation of multiplication of viable cancer cells or for bypassing the effects of a delta-6-desaturase deficiency comprising one or more precursors for the production in vivo of one or more of the following eicosanoid mixtures, namely

| a | b | c |
|---|---|---|
| $PGE_1$ | $PGE_2$ | $PGE_3$ |
| $PGF_1$ | $PGF_2$ | $PGF_3$ |
| $TXA_1$ | $PGD_2$ | $PGD_3$ |
| | Prostacyclin | $\Delta^{17}$Prostacyclin |
| | $TXA_2$ | $TXA_3$ |
| $LTA_3$ | $LTA_4$ | $LTA_5$ |
| $LTC_3$ | $LTB_4$ | $LTB_5$ |
| $LTD_3$ | $LTC_4$ | |
| | $LTD_4$ | |
| | $LTE_4$ | |

According to a further aspect of this invention there is provided a prophylactic composition comprising gamma linolenic acid (GLA) anachidonic acid (AA) and/or eicosapentaenoic acid (EPA), or salts, derivatives or analogues thereof.

BAD ORIGINAL

The present invention is concerned mainly with the prophylactic use of the substances for cancer and does not include the treatment of an already manifest pathological cancer. Generally speaking, the conventional cancer treatments, even if they could be used prophylactically (in small doses) cause extreme discomfort and distress and could even precipitate other cancerous conditions.

As opposed to the hypotheses of Horrobin tht GLA merely restores the ratio between $PGE_1$ and $TXA_2$ in malignant cells, it has now been found that the proliferation suppressive effect of GLA (and AA and EPA) depends on their ability to give rise to the production of a number of eicosanoids and related compounds; for example, $PGA_1$ and $PGD_1$ have a much greater proliferation suppressive effect on human cancer cells than does $PGE_1$. $PGB_1$ has a lesser effect whereas yet other PGs derived from GLA, including $PGF_{1a}$ and $PGF_{2a}$ have no effect at all on the proliferation of human cancer cells.

In a preferred form of the invention GLA, and/or AA and/or EPA is administered as a dietary supplement. The composition may be presented in a number of possible forms such as, for example, capsules, pills, tablets or other conventional pharmaceutical forms, or in a mixture with foodstuffs, beverages and the like. The unit doses, in the form of capsules, pills, tablets or dietary supplements may be packaged in a single pack. The amount of active ingredient in such supplement or foodstuff or beverage should preferably be no more than for an average daily intake of up to 100mg thereof per 100 kg body weight.

The invention may also provide a method of bypassing the effects of a delta-6-desaturase deficiency in otherwise healthy individuals.

The d-6-d may be stimulated by the inclusion of relatively large quantities of w-6-linolenic acid, or w-3-linolenic acid in the compositions of the invention.

It will be appreciated that suitable salts, derivatives or chemical analgoues of the above substances are also in the scope of the present invention. In particular the magnesium and zinc salts are important.

The substance or composition may be provided in unit dosage form, eg for daily or twice-daily administration, such as in tablets or capsules. In each capsule the active ingredient may be in solution, or it may be in the form of a tablet or particulate mixture, comprising the active ingredient together with a solid diluent or carrier. A unit dosage for daily administration typically for a person of 50 to 100 kg body weight, may contain up to 100 mg of active ingredient, which is 5 - 10% of the dose recommended for treatment of pathological cancer which is a difference in the order of magnitude.

Such a daily unit dosage is not useful for the treatment of pathological cancerous conditions. The preferred dosage of this invention is also very much lower than that used for treatment of alcholism and other diseases.

Suitable solvents for the active ingredients comprise plant oils, isotonic saline solutions or any other lipid or aqueous solvents suitable for human intake. The compositions of this invention may also contain Vitamin A derivatives (for protective effects against skin cancer) Vitamin B Complex (as co-factors), Vitamin C (with which there seems to be a synergistic effect), or Vitamin E (which also serves as an anti-oxidant). Zinc or magnesium salts may be added to the composition.

The invention will now be described and illustrated by way of the following examples; with reference to the accompanying drawings which are all graphical figures illustrating experimental results.

EXAMPLE I

Experimental Procedure

Materials

Gamma-linolenic acid (GLA)

The fatty acid GLA (Sigma L-2378) was prepared for addition to a culture medium under anaerobic conditions by the method described by Ingerman-Wojenski et al (Prostaglandins 1981 : 21, 655-664). The chemically

pure fatty acid was dissolved in 0,1M $Na_2CO_3$ to a concentration of 10 mg/ml, under $N_2$. This stock solution was stored at -80°C. When required, this stock mixture was further diluted with 0.1M $Na_2CO_3$ to final fatty acid concentrations ranging from 0.5 to 10 µg/ml. These final concentrations were added to the culture medium described hereunder, as required and after sterilisation by millipore filtration (type GS, pore size 0.22 µm).

Cells

Two cells lines were maintained in Greiner plastic flasks (Labortechnik) in McCoys 5A medium (Flow Laboratories) containing 10% fetal calf serum (Flow Laboratories) and antibiotics (CRYSTAPEN, ie sodium benzyl penicillin, and NOVOSTREP). Stock cultures were re-fed twice a week and sub-cultured at weekly intervals or sooner if required, ie when the culture became depleted. The two cell lines comprised BL6 mouse melanoma cells which were obtained from Dr. C. Albrecht, Department of Pharmacology, University of Stellenbosch, South Africa and MDBK bovine kidney cells which were obtained from Dr. D. Verwoerd, Veterinary Research Institute, Onderstepoort, South Africa.

The Critical Experiment

GLA dose response curves

Sixteen independent groups of dose response curves to GLA were evaluated by the following two standard procedures, ie:
(i) by determining the total cell count and viability (n = 6), and (ii) by assessing cell proliferation by determining the rates of DNA and protein synthesis, and using the incorporation of radioactive thymidine, (n = 5) and methionine (n = 5), respectively. All experiments were performed by the identical and simultaneous addition of the GLA to both cell lines, making a total of 32 separate experimental culture sets. Each of these 32 culture sets comprised two control cultures and a range of GLA-containing cultures. The GLA dosages (single dosage in each case) for the cell count procedure were 0.5; 1; 2; 3; 4; 5; 6; 7; 8; 9, and 10 µg/ml, respectively. Thus each culture set comprised 13 separate experiments. A total of 156 separate cultures were therefore used for the GLA growth rate determinations. The GLA dosages for both the thymidine and methionine were two controls

(without GLA), and 2, 3, 6, 8 and 10 µg/ml. Each set of dosages was duplicated for both cell lines. Thus, a total of 280 separate cultures were used for these determinations. For statistical treatment an average value of each duplicate was used as n = 1.

(i) Growth Curves Protocol: The cells were seeded in a standard growth medium (McCoys 5A medium) from stock cultures into Greiner 50 ml plastic flasks at subconfluent densities ranging from $1 \times 10^5$ cells/flask. Following cell attachment after 6 to 24 hours, GLA was added to give the final concentrations described above.

The control cultures were treated identically except for the omission of GLA. These cultures flasks were incubated in a humidified atmosphere of 5% $CO_2$, 95% air at 37°C for 7 days. After this period cell counts and viabilities were determined, the latter by trypan blue dye exclusion.

(ii) Cell Prolliferation Protocol: For the determination of DNA and protein synthesis 200 µl of cell suspension ($2.4 \times 10^4$ cells) were added to each of 12 wells of a Dynatech (Microtiter) plate (M 29 AR), followed, after cell attachment, by the GLA concentrations described above. Plates were incubated in a 5% $CO_2$ humidified atmosphere at 37°C. For the final 24 hours, 0.4 µCi of [6-$^3$H]-thymidine or 1 µCi of L-[methyl -$^3$H] -methionine (New England Nuclear, Boston, Mass) was added to each incubation mixture on the micrplate. The cells were then collected using a Skatron automatic harvester onto glass fibre reinforced resin filters. The filters were dried and dissolved in 10 ml Picofluor TM30 (Packard). The [6- $^3$H] -thymidine incorporation into DNA and the L-[methyl -$^3$H] -methionine incorporation into proteins was counted on a Packard Tri-Carb scintillation counter.

Data Reduction and Statistical Analyses: Using the values of cell counts obtained as described above, the difference of each cell count from its control was expressed as a perecentage of the control. As the growth curves were repeated several times for each dose for both normal and cancer cells, the mean value and standard error was determined for each dose. The statistical significance of the difference between the response of the BL6 and MDBK cells to GLA was determined using the paired Student t-test. For the purpose of presentation these percentage differences are

expressed as the percentage reduction of growth rate (-GR%).

Results

GLA Dose Response Curves

(i) Growth Rates: Figure I and Table I shows the percentage reduction of growth rates (-GR%) of BL6 and MDBK cells in response to increasing doses of GLA. A statistically highly significant percentage reduction in the growth rate of the BL6 cancer cells was found relative to the normal MDBK cells with every dose of GLA from 0.05 μg/ml (p 0.01) to 10 μg/ml (p 0.001). The dose response curve of the BL6 cancer cells showed a stepwise progression of the -GR% for GLA doses from 0.5 μg/ml to about 7.0 μg/ml. At GLA doses above 7 μg/ml the growth response curve reaches a plateau at a -GR% of about 70%. This is suggestive of a saturable dose response curve. The response of the MDBK cells is remarkable as there is no apparent response to any of the GLA doses.

(ii) Cell Proliferation: The effect in increasing GLA doses on the uptake of radioactive thymidine and methionine by BL6 and MDBK is shown in Table II and in Figures 2 and 3. As expected, these responses are similar to the respective cell growth responses. A statistically highly significant reduction in the uptake of methionine and thymidine occurred in the malignant BL6 cells as compared to the normal MDBK cells. At a GLA dose level of 10 ug/ml the reduction in methionine and thymidine uptake by BL6 cells amounted to 58.9% and 59.1% respectively.

TABLE I

The effect of increasing GLA doses on the growth rate of malignant BL6 and normal MDBK cells

| GLA concentration µg/ml | BL6 | | MDBK | | p value | Significance |
|---|---|---|---|---|---|---|
| | Mean growth rate. % reduction of control (-GR%) | ± Standard error of mean | Mean growth rate. % reduction of control (-GR%) | ± Standard error of mean | | |
| 0,5 | 16,42 | 3,69 | -3,23 | 3,46 | <0,01 | highly significant |
| 1,0 | 27,35 | 5,95 | -2,04 | 5,49 | <0,01 | highly significant |
| 2,0 | 34,87 | 6,57 | -1,56 | 7,27 | <0,01 | highly significant |
| 3,0 | 50,87 | 9,08 | 2,04 | 4,68 | <0,001 | highly significant |
| 4,0 | 54,3 | 6,39 | 3,3 | 5,89 | <0,001 | highly significant |
| 5,0 | 54,68 | 10,75 | -0,69 | 3,21 | <0,001 | highly significant |
| 6,0 | 60,08 | 6,16 | 3,16 | 2,96 | <0,001 | highly significant |
| 7,0 | 68,23 | 3,62 | -2,7 | 1,81 | <0,001 | highly significant |
| 8,0 | 64,12 | 1,72 | 1,43 | 6,4 | <0,001 | highly significant |
| 9,0 | 70,08 | 4,55 | 2,1 | 4,31 | <0,001 | highly significant |
| 10,0 | 68,43 | 7,04 | 3,13 | 4,56 | <0,001 | highly significant |

BAD ORIGINAL

TABLE III

he effect of increasing GLA doses on the uptake of radioactive thymidine and methionine by malignant BL6 nd normal MDBK cells

| GLA concentration µg/ml | BL6 | | MDBK | | p value | Significance |
|---|---|---|---|---|---|---|
| | Mean reduction in uptake (%) | ± Standard error of mean | Mean reduction in uptake (%) | ± Standard error of mean | | |
| | Thymidine uptake | | | | | |
| 2 | 34,85 | 5,19 | -16,53 | 10,51 | < 0,01 | highly significant |
| 4 | 42,42 | 8,25 | -11,97 | 11,08 | < 0,01 | highly significant |
| 6 | 45,69 | 4,73 | -17,79 | 9,87 | < 0,01 | highly significant |
| 8 | 46,34 | 7,62 | -21,8 | 6,15 | < 0,01 | highly significant |
| 10 | 59,09 | 8,44 | - 7,3 | 12,34 | < 0,01 | highly significant |
| | Methionine uptake | | | | | |
| 2 | 25,93 | 7,97 | -25,08 | 11,82 | < 0,01 | highly significant |
| 4 | 44,05 | 8,91 | -11,78 | 8,53 | ≤ 0,01 | highly significant |
| 6 | 51,94 | 8,97 | -19,11 | 13,35 | < 0,01 | highly significant |
| 8 | 55,65 | 9,11 | - 3,93 | 10,28 | < 0,01 | highly significant |
| 10 | 58,88 | 3,03 | - 1,49 | 7,26 | < 0,01 | highly significant |

BAD ORIGINAL

## EXAMPLE 2

### Experimental Procedure

### Materials

GLA: GLA (Sigma - 2378) was dissolved in sodium carbonate under $N_2$ as described above in Example 1. A GLA dose of 20 µg/ml was used throughout these experiments. This was achieved by adding 20 µl of GLA in sodium carbonate stock (10 mg/ml) directly to each GLA flask containing 10 ml of culture medium. To control flasks only 20 µl of the sodium carbonate vehicle was added.

Cells: Cells of the human primary carcinoma of the liver line were provided by Dr. J.J. Alexander of the Division of Virology, Department of Microbiology, Medical University of South Africa. These cells were maintained in Grenier plastic flasks in McCoys 5A medium as described in Example 1.

### Growth Rate Protocol

The cells were seeded in the standard growth medium from stock cultures into Greiner 50 ml plastic flasks at subconfluent densities ranging from $2 \times 10^5$ to $3 \times 10^5$ cells per flask. Following cell attachment after 6 hours, 20 µg/ml of GLA in sodium carbonate was added to the GLA flasks while sodium carbonate only was added to the control flasks. These culture flasks were included in a humidified atmosphere of 5% $CO_2$, 95% air at $37^o$. At the end of each experiment the detached, dead cells were discarded with the culture medium. The living attached cells were trypsinised and then counted using a Coulter counter. Cell viability was checked by trypan blue dye exclusion. In addition, the culture flasks were viewed and photographed daily under a Nikon diaphot inverted phase microscope. To determine the effect of the duration of GLA treatment, two different procedures were adopted.

(i). Daily Dose effect: The culture medium was changed daily and new GLA was added every day. Cells were photographed and counted on day 1, day 2, day 3, and day 4 (run 1: n = 4 for both GLA and controls on each day). The procedure for day 4 was repeated: (run 2: n = 4 for both GLA and controls).

(ii) Single dose effect: One dose of GLA was added to the GLA flasks (n = 8) and one dose of sodium carbonate to the controls (n = 8) on day 1 of the experiment. The cells were counted after 4 days in culture.

Results

Daily Dose Effect

Table III shows the effects of a daily dose of GLA on the growth rate of human hepatoma cells. The average reduction of the growth rate in the GLA flasks compared to the control flasks was 66% on day 2, 78% on day 3, and 87% on day 4 for run 1 and run 2 respectively. The mean values and standard error of mean for run one is shown in Figure 4. This shows the normal proliferation rate of the untreated control cells. There was a plating efficiency of about 35% and a doubling every two days, which has been reported to be typical for this hepatoma cell line. In contrast, there was a statistically highly significant reduction in the growth rate of the GLA treated cells when compared with the control cells. The following differences were noted after 3 days between the treated GLA cells and the control cells at magnifications of 100x and 200x. The control cells were uniformly distributed over the flask. In contrast, there were large areas with zero cell growth in the GLA treated flasks and the visualised cells were far fewer, less dense and differed markedly from both the controls. This was reported as typical for this cell line.

Single Dose Effects:

After four days, the cell count, after a single dose of GLA on day 1 was $19.81 \times 10^4 \pm 4.85$ compared to $21.8 \times 10^4$ for the controls. There was no significant difference between the growth rates of GLA supplemented and control cells.

The results of Examples 1 and 2 show that for both the mouse melanoma cells and the human cancer cells the in vitro supplementation of GLA produces a statistically significant reduction in the growth rate of the cancerous cells. Thus, by maintaining the level of GLA at a certain level the uncontrolled multiplication of potential cancer cells is prevented.

TABLE III

The effect of daily gamma-linolenic acid supplementation on the growth rate of human hepatoma cells in culture

| Day of experiment | GLA supplemented cell number ($x10^4$) | | Control cell number ($x10^4$) | | Reduction in growth rate (%) | p value | Significance |
|---|---|---|---|---|---|---|---|
| | Mean n = 4 | ± SE of mean | Mean n = 4 | ± SE of mean | | | |
| 1 | 8,12 | 0,88 | 5,63 | 1,08 | -44 | $>$ 0,05 | non significant |
| 2 | 3,79 | 0,39 | 11,25 | 0,73 | 66 | $<$ 0,01 | highly significant |
| 3 | 3,35 | 0,29 | 15,42 | 0,87 | 78 | $<$ 0,01 | highly significant |
| 4 Run 1 | 2,96 | 0,47 | 23,44 | 1,83 | 87 | $<$ 0,01 | highly significant |
| 4 Run 2 | 7,25 | 0,75 | 32,5 | 1,96 | 78 | $<$ 0,01 | highly significant |

BAD ORIGINAL

In contrast to the mouse melanoma cell response to a single dose of GLA, the human hepatoma cells appeared to require repeated supplementation of GLA to produce a similar effect.

EXAMPLE 3

Methods and materials

Cells: MG63 osteoid-sarcoma cells were obtained from Flow Laboratories. Oesophageal carcinoma cells were obtained from Prof. J. Alexander of the Department of Microbiology at Medunsa, South Africa. Control benign bovine kidney cells (MDBK) were from the same stock that were used in the previous examples.

Techniques: The techniques used for growing cells in culture, GLA supplementation of the growth media and for obtaining cell counts were similar to those reported in the previous examples.

Results

MG63 Osteoid-sarcoma cells: From Figure 5 it can be seen that GLA supplementation of the culture media of MG63 cells caused a marked, statistically highly significant growth suppressive effect of these cells compared with the growth of unsupplemented MG63 cells (Table IV): At the end of seven days in culture following daily GLA supplementation, the mean number of MG63 cells increased from the initial seeding density of $0.3 \times 10^6$ cells to $0.56 \times 10^6$.

The mean number of untreated control MG63 cells increased from $0.3 \times 10^6$ to $2.3 \times 10^6$ cells over the same period of time. This difference in growth rate represents a growth suppressive effect of 76% in the GLA supplemented MG63 cells over the seven days experimental period. The mean number of GLA supplemented control MDBK cells, in contrast to the MG63 cells, exceed the mean number of unsupplemented MDBK cells at the end of the seven day period.

Oesophageal carcinoma cells: Figure 6 shows the growth rate of oesophageal carcinoma cells following supplementation of their growth media with 10 µg GLA per day for six days, compared to the growth of control cultures of unsupplemented oesophageal carcinoma cells. The mean

number of unsupplemented cells increased from the initial seeding number of $0.14 \times 10^6$ to $6.62 \times 10^6$ at the end of eight days in culture (Table IV). In contrast, the mean number of GLA supplemented cells increased from $0.14 \times 10^6$ to only $2.04 \times 10^6$ cells over the same period of time. This growth suppressive effect of 69% at the end of the eight day period was found to be statistically highly significant. A statistically significant growth suppressive effect by GLA of the oesophageal carcinoma cells became evident from the sixth day in culture (Table IV).

EXAMPLE 4

Experimental Procedure

Materials

GLA: GLA (Sigma - 2378) was dissolved in sodium carbonate under $N_2$ as described in Examples 1 and 2. A GLA dose of 20 μ l of the sodium carbonate stock contains the GLA was added directly to each flask containing 10 ml of the later described culture medium. Only 20 μ l of the sodium carbonate vehicle was added to control flasks.

Cells.

Malignant BL6 mouse melanoma cells were maintained in Griener plastic flasks (Labortechnik) containing 10% fetal calf serum (Flor Laboratories) and antibiotics. Stock cultures were refed twice weekly and were subcultured at weekly intervals or sooner if required. The BL6 mouse melanoma cells were supplied by Dr. C. Albrecht, Department of Pharmacology, University of Stellenbosch, South Africa.

Procedure

BL6 melanoma cells were seeded in the standard growth medium from stock cultures into 50 ml Greiner plastic flasks to give subconfluent densities of 500, 1,000 and 2,000 cells/flasks. Duplicates were set up for the three densities. Following cell attachment after 24 hours, GLA was added to the growth media in the flasks to give the final concentration of 20 μg/ml.

Growth media were replaced daily and GLA was added daily for two

weeks. Control cultures were treated identically except for the omission of GLA. The culture flasks were incubated in a humidified atmosphere of 5% $CO_2$, 95% air at 37°C. At the end of the two weeks period the culture flasks were stained using a 0.1% Amidoschwarz stain solution.

Results

At the end of the two weeks the initially seeded melanoma cells had established colonies of various sizes in the control culture flasks. The densest concentration of colonies were formed in the flasks in which 2,000 cells were seeded. In contrast, not a single colony could be observed after two weeks in the GLA treated culture flasks, in either the 500, 1,000 or 2,000 cells seeded flasks. The growth and proliferation of the malignant BL6 melanoma cells was therefore completely suppressed by GLA supplementation.

The results of this experiment are clear-cut and two facts become evident from the results: Firstly, they confirm that the malignancy in this particular cell type is GLA deficiency dependent. Secondly, the growth of such malignant cells can be completely suppressed by the presence of GLA.

0106571

TABLE IV : The effect of daily GLA supplementation on the rate of growth of benign MDBK, MG63 osteoid-sarcoma and human oesophageal cells in culture

| Cell type and seeding density (x $10^6$) | Day in culture | Cell count (n = 4) (x $10^6$) | | | | | |
|---|---|---|---|---|---|---|---|
| | | Controls | | + GLA | | | |
| | | Mean | SE | Mean | SE | p | Sign |
| MDBK 0,3 | 2 | 0,41 | 0,02 | 0,43 | 0,02 | > 0,05 | ns |
| | 3 | 0,76 | 0,003 | 0,70 | 0,02 | 0,05 < > 0,01 | ps |
| | 6 | 1,2 | 0,04 | 1,53 | 0,06 | < 0,01 | hs |
| | 7 | 1,3 | 0,05 | 1,81 | 0,01 | < 0,01 | hs |
| MG63 0,3 | 2 | 0,21 | 0,02 | 0,19 | 0,008 | < 0,05 | ns |
| | 3 | 0,41 | 0,04 | 0,31 | 0,007 | 0,05 < > 0,01 | ps |
| | 4 | 0,69 | 0,07 | 0,48 | 0,018 | 0,05 < > 0,01 | ps |
| | 7 | 2,34 | 0,14 | 0,56 | 0,034 | < 0,01 | hs |
| Oesophageal Carcinoma 0,14 | 2 | 0,18 | 0,03 | 0,14 | 0,02 | > 0,05 | ns |
| | 3 | 0,22 | 0,01 | 0,22 | 0,01 | > 0,05 | ns |
| | 4 | 0,39 | 0,05 | 0,31 | 0,01 | > 0,05 | ns |
| | 5 | 1,07 | 0,16 | 0,68 | 0,10 | > 0,05 | ns |
| | 6 | 2,39 | 0,19 | 0,63 | 0,12 | < 0,01 | hs |
| | 7 | 4,22 | 0,28 | 1,82 | 0,21 | < 0,01 | hs |
| | 8 | 6,62 | 0,35 | 2,04 | 0,19 | < 0,01 | hs |

BAD ORIGINAL

EXAMPLE 5

Human osteogenic sarcoma cells were seeded into 50 ml Greiner plastic flasks and maintained exactly as explained in Example 4.

The cells were cultured for three weeks in the presence of

i) growth medium only - control

ii) growth medium & 10 μl $Na_2CO_3$/ml of medium added every second day

iii) growth medium & 20 μg AA/ml of medium added every second day

iv) growth medium & 20 μg Linoleic acid/ml of medium added every second day

v) growth medium & 20 μg Oleic acid/ml of medium added every second day

vi) growth medium & 20 μg GLA/ml of medium added every second day

vii) growth medium & 5 μg $PGE_1$/ml of medium added every second day

viii) growth medium & 5 μg $PGA_1$/ml of medium added every second day

ix) growth medium & 5 μg $PGF_{1\alpha}$/ml of medium added every second day

At the end of three weeks the culture flasks were stained with a 0.1% Amidoschwarz stain solution.

RESULTS

At the end of the three weeks period the initially seeded osteogenic sarcoma cells had established colonies of various sizes almost covering the entire floor of the culture flasks of the control and $Na_2CO_3$ supplemented flasks.

Linoleic acid supplemented cultures achieved about 75% of the growth of control cultures. Oleic acid supplemented cultures achieved about the same growth as control cultures.

$PGE_1$ and $PGA_1$ cultures achieved about 25% of the growth of control cultures.

$PGF_{1\alpha}$ cultures achieved about the same growth as the control cultures.

GLA and AA supplemented cultures were completely devoid of any colonies in 500, 1,000 and 2,000 cell density cultures.

The results show that oleic acid has no suppressive effect on cancer cells and that the prostaglandin precursor fatty acid linoleic acid had some growth suppressive effect on the cancer cells in culture (because of its stimulative effect on d-6-d). $PGE_1$ and $PGA_1$, had a more pronounced growth suppressive effect whilst $PGF_{1\alpha}$ had no effect at all on cancer cell growth. In contrast, GLA & AA, the intermediates between the precursor fatty acids and the prostaglandins had a complete growth suppressive effect.

The results would suggest that uncontrolled cell division in cancer cells could be the result of abnormalities in the concentration of some of the prostaglandins in such cells, resulting from a block in their synthesis from precursor fatty acids. Such abnormalities are evidently eliminated by supplying the cancer cells with GLA or AA, the substrate from which the required prostaglandins can be synthesized in their required concentrations. Once this can be achieved by cancer cells, their uncontrolled division is apparently totally checked.

EXAMPLE 6

In view of the surprising observation reported in Example 5, that arachidonic acid (AA) supplementation at a rate of 40 μl/ml medium of mg63 osteogenic sarcoma cells completely suppresses proliferation and colony formation of the cells in culture, this experiment was repeated in order to confirm the observation. In addition, the effect of cancer cell

supplementation with eicosapentaenoic acid (SPA) the essential fatty acid intermediate prostaglandin precursor of the 3-series prostaglandins, prostacyclin, thromboxane A3 and leukotrienes, was also investigated. EPA is derived from the naturally occurring essential fatty acid A-linolenic acid (C 18:3 W3) by the action of d-6-d to give octadecatetraenoic acid (C 18:4 W3), which undergoes chain elongation to eicosatetraenoic acid (C20:4 W3), which in turn gives rise to eicosatetraenoic acid following the action of delta-5-desaturase.

PROCEDURE

MG63 Human osteogenic sarcoma cells were seeded in culture flasks as described in Examples 4 and 5. 2,000 cells were seeded in each flask. Duplicate sets of flasks were used for each of the fatty acids tested. The following fatty acids dissolved in standard growth medium were added to the cells in culture, after allowing 2 days for cell attachment, and again after a further 3 days. Each culture therefore had only 2 additions of the relevant fatty acid. The cells were stained and examined at the end of 7 days in culture:

1. Culture medium only - control.

2. 5, 10, 20, 40, 60, 80 and 100 μg oleic acid respectively /ml culture medium. (Oleic acid (OA) is an 18C fatty acid with one unsaturated bond in the omega-9 position. It is therefore structurally nearly identical to either linoleic acid (LA) and a-linolenic acid (ALA) with the singular exception of the number of double bonds contained in the molecule. On account of the latter differences, OA, unlike LA and ALA cannot give rise to eicosanoids (PG'S, LT.S, TXA'S and prostacyclin). It is therefore considered to be an excellent fatty acid to use as a control when investigating the effects of the eicosanoids).

3. 5; 10, 20, 40, 60, 80 and 100 μg LA respectively /ml culture medium.

4. 5, 10, 20, 40, 60, 80 and 100 μg ALA respectively /ml culture medium.

5. 5, 10, 20, 40, 60, 80 and 100 μg/ml AA respectively /ml culture medium.

6. 5, 10, 20, 40, 60, 80 and 100 μg/ml EPA respectively /ml culture medium.

7. 5, 10, 20, 40, 60, 80 and 100 μg/ml GLA respectively /ml culture medium.

RESULTS

1. OA supplemented cells achieved equal densities of colonies for all levels of supplementation between 5 and 100 μg/ml, and as did the controls with culture medium only.

2. LA and ALA had a definite proliferation suppressive action at the higher levels of supplementation, that is at 80 and 100 μg/ml.

3. EPA, AA and GLA exhibited an almost equal, progressive, suppressive action on the proliferation and colony formation of the MG63 osteogenic carcoma cells.

4. EPA, AA and GLA completely suppressed cell growth and colony formation at levels of supplementation above 40 μg/ml culture medium.

Not a single cell or colony of cells could be found on microscopic examination of the cultures which had been supplemented with either EPA, AA or GLA respectively at supplementation levels between 40 and 100 μg/ml.

It would therefore appear that the three precursor fatty acid intermediates, GLA, AA and EPA, which each gives rise to a separate family of eicosanoids, have the ability to individually arrest and suppress cancer cell growth. It would further appear that any one of these eicosanoid precursor fatty acids separately or in combination could be used prophylactically against cancer.

The features disclosed in the foregoing description, in the following claims and/or in the accompanying drawings may, both separately and in combination thereof, the material for realising the invention in diverse forms thereof.

CLAIMS:

1. A prophylactic method for the prevention of the multiplication of viable cancer cells including the step of administering on a regular basis to a human or an animal a prophylactic amount of one or more precursors for the production in vivo of one or more of the following eicosanoid mixtures, namely

| a | b | c |
|---|---|---|
| $PGE_1$ | $PGE_2$ | $PGE_3$ |
| $PGF_1$ | $PGF_2$ | $PGF_3$ |
| $TXA_1$ | $PGD_2$ | $PGD_3$ |
| | Prostacyclin | $\Delta^{17}$Prostacyclin |
| | $TXA_2$ | $TXA_3$ |
| $LTA_3$ | $LTA_4$ | $LTA_5$ |
| $LTC_3$ | $LTB_4$ | $LTB_5$ |
| $LTD_3$ | $LTC_4$ | |
| | $LTD_4$ | |
| | $LTE_4$ | |

according to the following scheme:-

BAD ORIGINAL

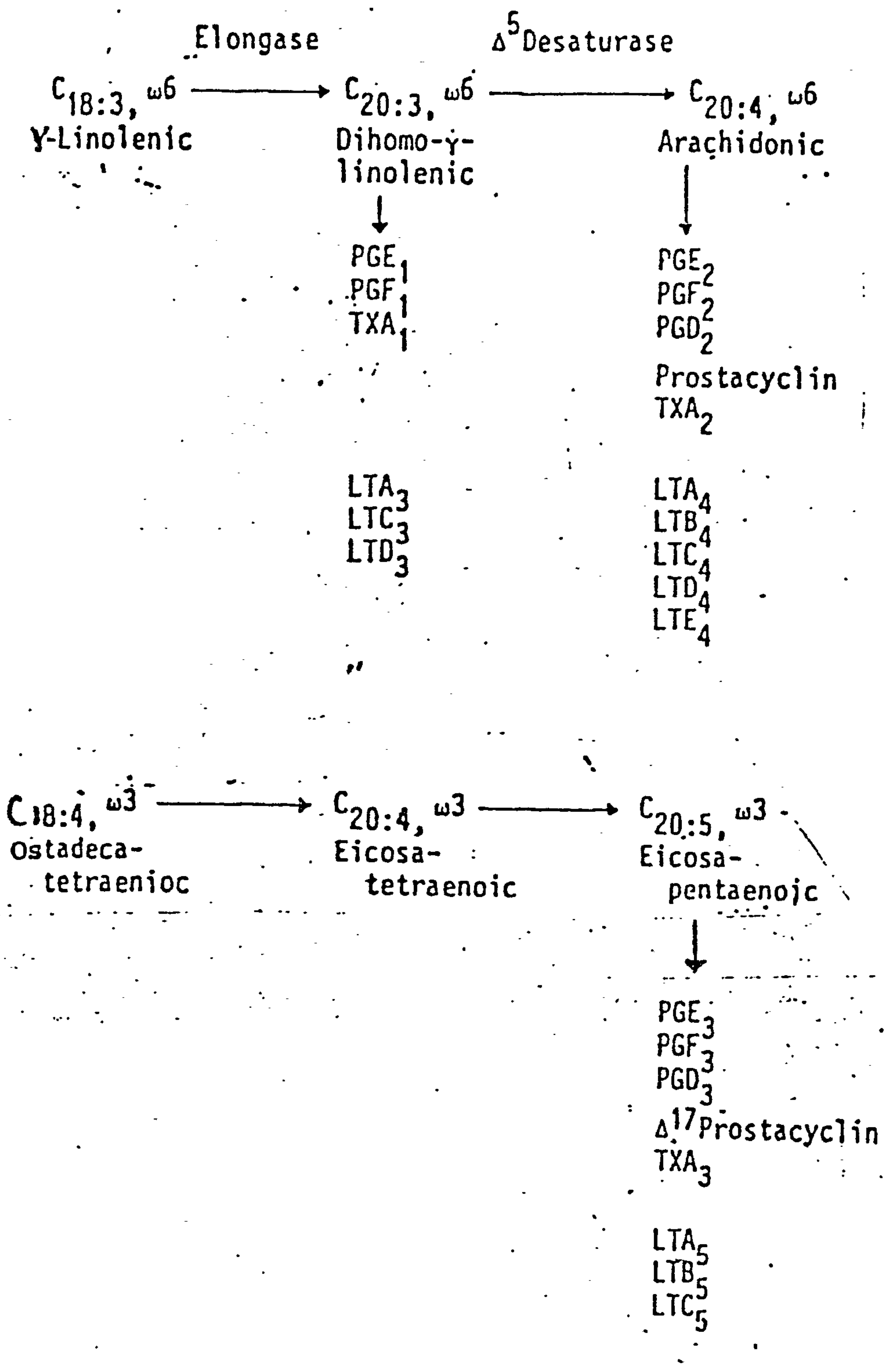


BAD ORIGINAL

2. The prophylactic method according to claim 1 in which the precursor is chosen from GLA, AA and/or EPA or salts, derivatives or analogues thereof.

3. A prophylactic composition including one or more of the precursors referred to in claim 2 in an amount for unit dosage form for daily administration of up to 100 mg of precursor per 100 Kg body mass of the patient.

4. A dietary supplement including one or more of the precursors chosen from GLA, AA and/or EPA or salts derivatives or analogues thereof for unit dosage form for daily administration of up to 100 mg of precursor per 100 Kg body mass of the patient.

5. A foodstuff or beverage including a dietary supplement according to claim 4.

6. A prophylactic composition for the prevention or minimisation of multiplication of viable cancer cells or for bypassing the effects of a delta-6-desaturase deficiency comprising one or more precursors for the production in vivo of one or more of the following eicosenoid mixtures, namely

| a | b | c |
|---|---|---|
| $PGE_1$ | $PGE_2$ | $PGE_3$ |
| $PGF_1$ | $PGF_2$ | $PGF_3$ |
| $TXA_1$ | $PGD_2$ | $PGD_3$ |
| | Prostacyclin | $\Delta^{17}$Prostacyclin |
| | $TXA_2$ | $TXA_3$ |
| $LTA_3$ | $LTA_4$ | $LTA_5$ |
| $LTC_3$ | $LTB_4$ | $LTB_5$ |
| $LTD_3$ | $LTC_4$ | |
| | $LTD_4$ | |
| | $LTE_4$ | |

BAD ORIGINAL

7. A prophylactic composition comprising gamma linolenic acid (GLA) anachidonic acid (AA) and/or eicosapentaenoic acid (EPA), or salts, derivatives or analogues thereof.

8. A prophylactic composition according to claim 7 in the form of unit doses constituted by capsules, pills or tablets.

9. A prophylactic composition according to claim 7 in the form of a unit dosage of a dietary supplement to be added to a foodstuff or beverage.

10. A prophylactic composition according to claim 8 or 9, which each unit dose contains less than 100 mg of active ingredient.

11. A prophylactic composition according to any one of claims 6 to 10 also containing vitamins, and/or derivatives or complexes thereof.

12. A prophylactic composition according to any one of claims 6 to 11 including W-6 linoleic acid and/or W-3 linoleic acid.

13. A prophylactic composition according to any one of claims 6 to 10 containing zinc or magnesium salts.

14. A foodstuff or beverage containing a dietary supplement according to claim 8 or any claim dependent thereon.

15. A package containing a plurality of unit doses of a prophylactic composition according to any one of claims 6 to 13.

1/5



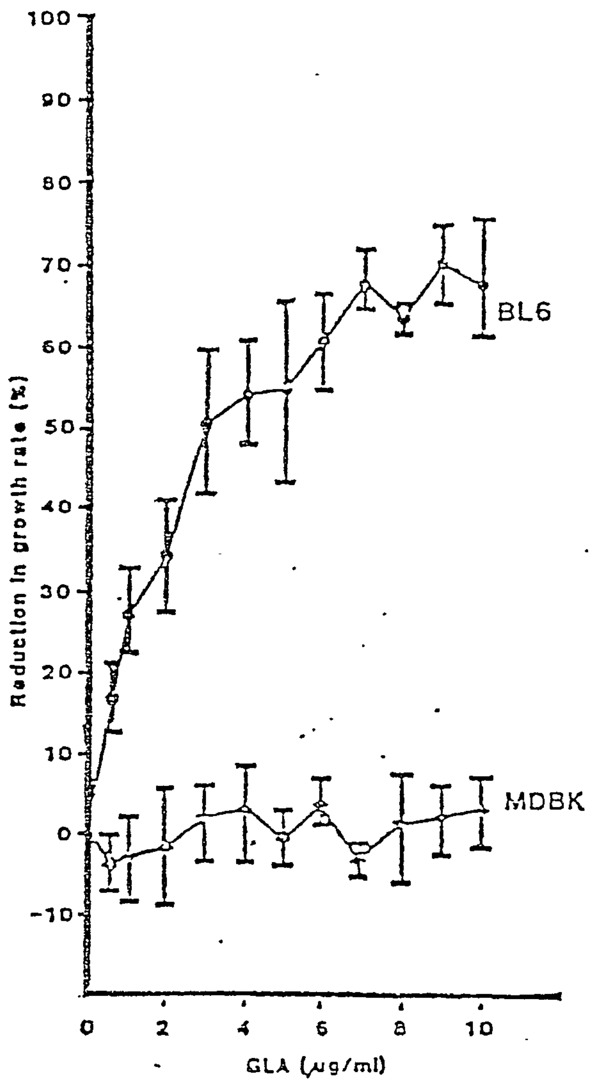


Fig.1 Reduction in growth rate (-GR%) of malignant BL6 and normal MDBK cells in response to increasing doses of GLA

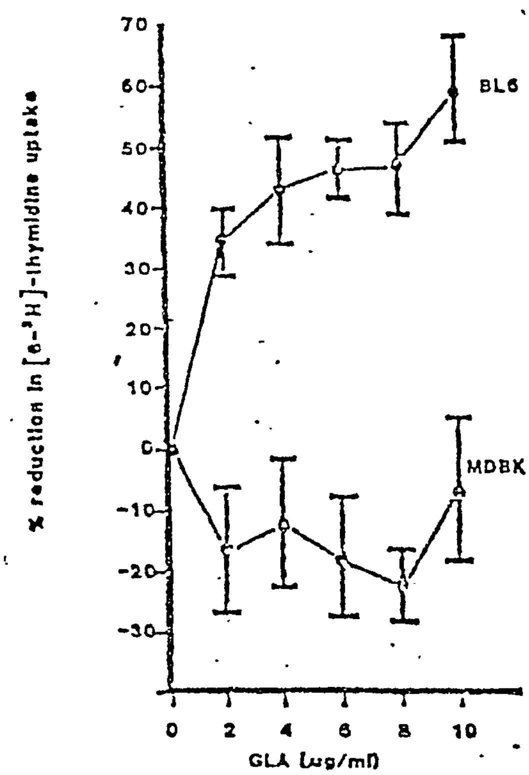


Fig.2 Comparison of radioactive thymidine uptake by malignant BL6 and normal MDBK cells in the presence of increasing GLA doses (expressed as the % reduction as compared to the control value)

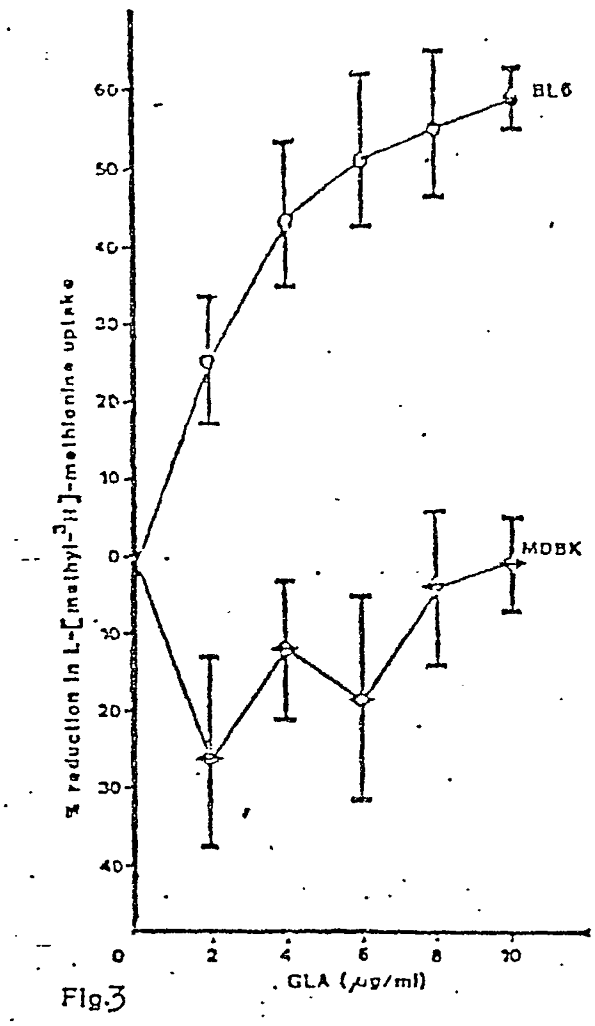


Fig.3

Comparison of radioactive methionine uptake between malignant BL6 and normal MDBK cells in the presence of increasing GLA doses (expressed as the % reduction as compared to the control value)

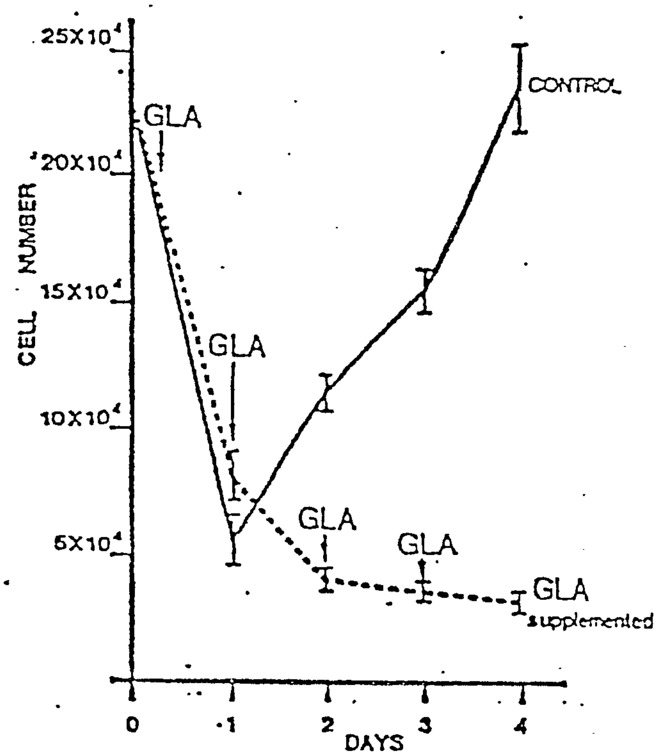


FIGURE 4 THE GROWTH RATE OF CULTURED HUMAN HEPATOMA CELLS IN THE PRESENCE AND ABSENCE OF GAMMA-LINOLENIC ACID

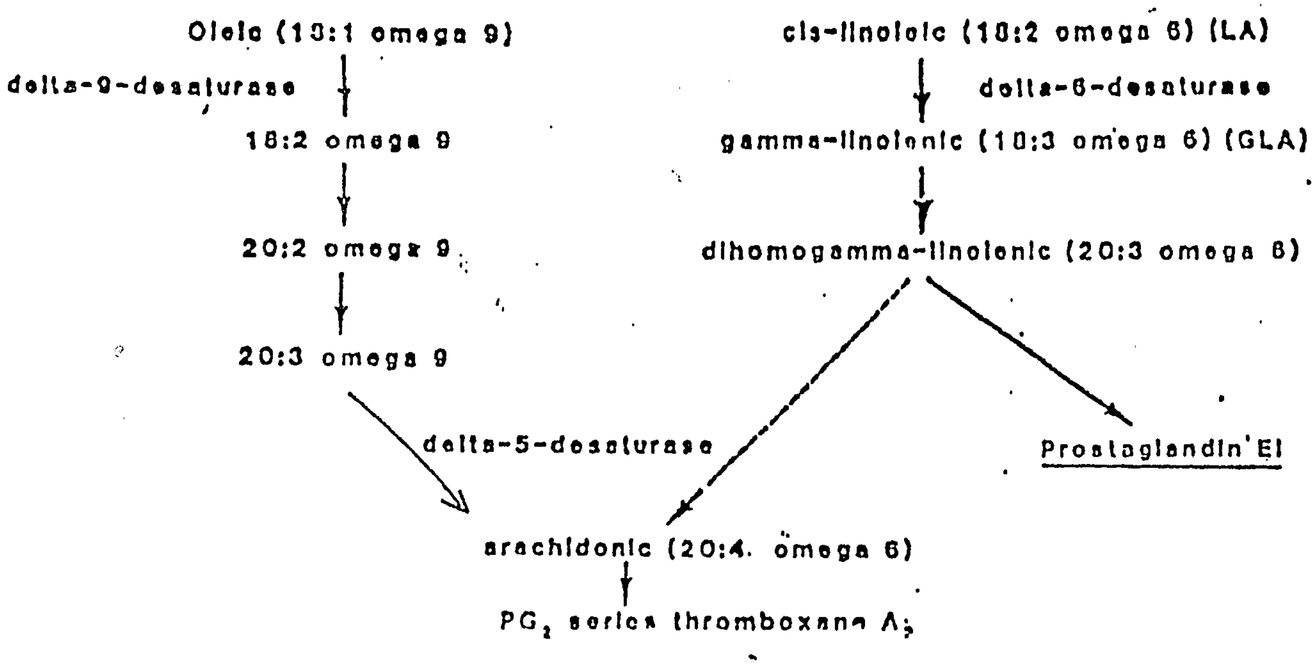


Fig.5 Synthesis of prostaglandins (Horrobin,1980)